(19) Europäisches Patentamt
European Patent Office
Office européen des brevets

(11) EP 3 431 089 A1

(12) **EUROPEAN PATENT APPLICATION**
published in accordance with Art. 153(4) EPC

(43) Date of publication:
**23.01.2019 Bulletin 2019/04**

(21) Application number: **17766642.7**

(22) Date of filing: **14.03.2017**

(51) Int Cl.:
***A61K 31/702*** *(2006.01)* ***A23L 33/125*** *(2016.01)*
***A61P 1/04*** *(2006.01)* ***A61P 3/04*** *(2006.01)*
***A61P 3/10*** *(2006.01)* ***A61P 35/00*** *(2006.01)*
***A61P 43/00*** *(2006.01)* ***C12N 1/20*** *(2006.01)*

(86) International application number:
**PCT/JP2017/010066**

(87) International publication number:
**WO 2017/159643 (21.09.2017 Gazette 2017/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **14.03.2016 JP 2016049561**

(71) Applicant: **B Food Science Co., Ltd.**
**Chita-shi**
**Aichi 478-0046 (JP)**

(72) Inventors:
- **TOCHIO, Takumi**
  **Chita-shi**
  **Aichi 478-0046 (JP)**
- **KONISHI, Kenta**
  **Chita-shi**
  **Aichi 478-0046 (JP)**
- **NAKAMURA, Saki**
  **Chita-shi**
  **Aichi 478-0046 (JP)**

(74) Representative: **Wasner, Marita**
**Niizuma Wasner GmbH**
**Patentanwaltskanzlei**
**Postfach 278**
**4125 Riehen 1 (CH)**

# (54) AGENT FOR INCREASING INTESTINAL BUTYRIC ACID AND PROLIFERATION AGENT FOR BUTYRIC ACID-PRODUCING BACTERIA

(57) To provide: an agent for increasing intestinal butyric acid which is capable of effectively increasing intestinal butyric acid; a food composition for increasing intestinal butyric acid; a proliferation agent for butyric acid-producing bacteria; a food composition for proliferating butyric acid-producing bacteria; a method for increasing intestinal butyric acid; a method for proliferating butyric acid-producing bacteria; a method for treating or preventing intestinal inflammation, fatty liver, diabetes, colorectal cancer or obesity using the same; and use of 1-kestose for producing a pharmaceutical preparation for treatment or prevention of these diseases.

[Solution]

An agent for increasing intestinal butyric acid and a proliferation agent for butyric acid-producing bacteria, each comprising 1-kestose as an active ingredient. According to the present invention, intestinal butyric acid in humans or animals can be easily and effectively increased with little side effects or safety concerns. According to the present invention, furthermore, intestinal inflammation, fatty liver, diabetes, colorectal cancer or obesity can be prevented or treated by increasing intestinal butyric acid in humans or animals.

[Figure 2]

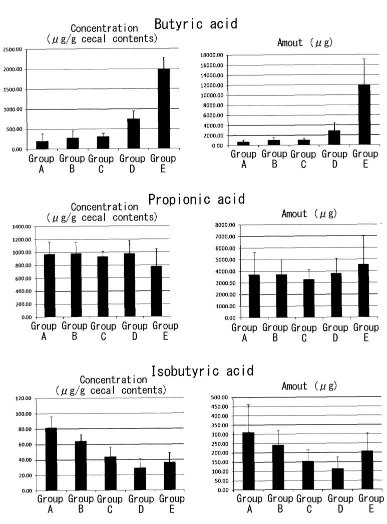


EP 3 431 089 A1

SEQUENCE LISTING

<110> B Food Science Co., Ltd.

<120> Intestinal butyric acid increaser and butyric acid-producing bacteria proliferator

<130> PCT0732

<150> JP2016-049561
<151> 2016-03-14

<160> 12

<170> PatentIn version 3.5

<210> 1
<211> 22
<212> DNA
<213> Artificial Sequence

<220>
<223> forward primer

<400> 1
gattctggct caggatgaac gc 22

<210> 2
<211> 20
<212> DNA
<213> Artificial Sequence

<220>
<223> reverse primer

<400> 2
ctgataggac gcgaccccat 20

<210> 3
<211> 20
<212> DNA
<213> Artificial Sequence

<220>
<223> forward primer

<400> 3
gawgaagtat ytcggtatgt 20

<210> 4
<211> 17
<212> DNA
<213> Artificial Sequence

<220>
<223> reverse primer

<400> 4
ctacgcwccc tttacac 17

<210> 5
<211> 22
<212> DNA
<213> Artificial Sequence

<220>
<223> forward primer

<400> 5
cacaatggac gmaagtctga tg 22

<210> 6
<211> 20
<212> DNA
<213> Artificial Sequence

<220>
<223> reverse primer

<400> 6
cgccactggt gttcttccat 20

<210> 7
<211> 20
<212> DNA
<213> Artificial Sequence

<220>
<223> forward primer

<400> 7
cagcacgtga aggtggggac 20

<210> 8
<211> 20
<212> DNA
<213> Artificial Sequence

<220>
<223> reverse primer

<400> 8
ccttgcggtt ggcttcagat 20

<210> 9
<211> 1507
<212> DNA
<213> Bifidobacterium longum subsp. longum JCM 1217T

<400> 9
gggtttcgat tctggctcag gatgaacgct ggcggcgtgc ttaacacatg caagtcgaac 60

gggatccatc aagcttgctt ggtggtgaga gtggcgaacg ggtgagtaat gcgtgaccga 120

cctgccccat acaccggaat agctcctgga aacgggtggt aatgccggat gttccagttg 180

atcgcatggt cttctgggaa agctttcgcg gtatgggatg gggtcgcgtc ctatcagctt 240

gacggcgggg taacggccca ccgtggcttc gacgggtagc cggcctgaga gggcgaccgg 300

ccacattggg actgagatac ggcccagact cctacgggag gcagcagtgg ggaatattgc 360

acaatgggcg caagcctgat gcagcgacgc cgcgtgaggg atggaggcct tcgggttgta 420

aacctctttt atcggggagc aagcgagagt gagtttaccc gttgaataag caccggctaa 480

ctacgtgcca gcagccgcgg taatacgtag ggtgcaagcg ttatccggaa ttattgggcg 540

taaagggctc gtaggcggtt cgtcgcgtcc ggtgtgaaag tccatcgctt aacggtggat 600

ccgcgccggg tacgggcggg cttgagtgcg gtaggggaga ctggaattcc cggtgtaacg 660

gtggaatgtg tagatatcgg gaagaacacc aatggcgaag gcaggtctct gggccgttac 720

tgacgctgag gagcgaaagc gtggggagcg aacaggatta gataccctgg tagtccacgc 780

cgtaaacggt ggatgctgga tgtggggccc gttccacggg ttccgtgtcg gagctaacgc 840

gttaagcatc ccgcctgggg agtacggccg caaggctaaa actcaaagaa attgacgggg 900

gcccgcacaa gcggcggagc atgcggatta attcgatgca acgcgaagaa ccttacctgg 960

gcttgacatg ttcccgacgg tcgtagagat acggcttccc ttcggggcgg gttcacaggt 1020

ggtgcatggt cgtcgtcagc tcgtgtcgtg agatgttggg ttaagtcccg caacgagcgc 1080

aaccctcgcc ccgtgttgcc agcggattat gccgggaact cacgggggac cgccggggtt 1140

aactcggagg aaggtgggga tgacgtcaga tcatcatgcc ccttacgtcc agggcttcac 1200

gcatgctaca atggccggta caacgggatg cgacgcggcg acgcggagcg gatccctgaa 1260

aaccggtctc agttcggatc gcagtctgca actcgactgc gtgaaggcgg agtcgctagt 1320

aatcgcgaat cagcaacgtc gcggtgaatg cgttcccggg ccttgtacac accgcccgtc 1380

aagtcatgaa agtgggcagc acccgaagcc ggtggcctaa ccccttgtgg gatggagccg 1440

tctaaggtga ggctcgtgat tgggactaag tcgtaacaag gtagccgtac cggaaggtgc 1500

ggctgga 1507

<210> 10
<211> 1493
<212> DNA
<213> Clostridium clostridioforme JCM 1291T

<220>
<221> misc_feature
<222> (1123)..(1124)
<223> n is a, c, g, or t

<400> 10
agagtttgat cctggctcag gatgaacgct ggcggcgtgc ctaacacatg caagtcgaac 60

gaagcaatta agatgaagtt ttcggatgga atcttgattg actgagtggc ggacgggtga 120

gtaacgcgtg gataacctgc ctcacactgg gggataacag ttagaaatga ctgctaatac 180

cgcataagcg cacagtgccg catggcagtg tgtgaaaaac tccggtggtg tgagatggat 240

ccgcgtctga ttagccagtt ggcggggtaa cggcccacca aagcgacgat cagtagccga 300

cctgagaggg tgaccggcca cattgggact gagacacggc ccaaactcct acgggaggca 360

gcagtgggga atattgcaca atgggcgaaa gcctgatgca gcgacgccgc gtgagtgaag 420

aagtatttcg gtatgtaaag ctctatcagc agggaagaaa atgacggtac ctgactaaga 480

agccccggct aactacgtgc cagcagccgc ggtaatacgt agggggcaag cgttatccgg 540

atttactggg tgtaaaggga gcgtagacgg cgaagcaagt ctgaagtgaa aacccagggc 600

tcaaccctgg gactgctttg gaaactgttt tgctagagtg tcggagaggt aagtggaatt 660

cctagtgtag cggtgaaatg cgtagatatt aggaggaaca ccagtggcga aggcggctta 720

ctggacgata actgacgttg aggctcgaaa gcgtggggag caaacaggat tagataccct 780

ggtagtccac gccgtaaacg atgaatgcta ggtgttgggg ggcaaagccc ttcggtgccg 840

ccgcaaacgc agtaagcatt ccacctgggg agtacgttcg caagaatgaa actcaaagga 900

attgacgggg acccgcacaa gcggtggagc atgtggttta attcgaagca acgcgaagaa 960

ccttaccaag tcttgacatc cccctgacgg gccggtaacg cggcctttcc ttcgggacag 1020

gggagacagg tggtgcatgg ttgtcgtcag ctcgtgtcgt gagatgttgg gttaagtccc 1080

gcaacgagcg caacccttat ccttagtagc cagcasgtar agnngggcac tctagggaga 1140

ctgccaggga taacctggag gaaggtgggg atgacgtcaa atcatcatgc cccttatgat 1200

ttgggctaca cacgtgctac aatggcgtaa acaaagggaa gcgagacagt gatgtggagc 1260

aaatcccaaa aataacgtcc cagttcggac tgtagtctgc aacccgacta cacgaagctg 1320

gaatcgctag taatcgcgaa tcagaatgtc gcggtgaata cgttcccggg tcttgtacac 1380

accgcccgtc acaccatggg agtcagcaac gcccgaagtc agtgacccaa ccgaaaggag 1440

ggagctgccg aaggcggggc aggtaactgg ggtgaagtcg taacaaggta acc 1493

<210> 11
<211> 1522
<212> DNA
<213> Lactobacillus casei JCM 1134T

<400> 11
gatgaacgct ggcggcgtgc ctaatacatg caagtcgaac gagttttggt cgatgaacgg 60

tgcttgcact gwgattcrac ttaaaacgag tggcggacgg gtgagtaaca cgtgggtaac 120

ctgcccttaa gtgggggata acatttggaa acagatgcta ataccgcata aatccaagaa 180

ccgcatggtt cttggctgaa agatggcgtc aagctatcgc ttttggatgg acccgcggcg 240

tattagctag ttggtgaggt aacggctcac caaggcgatg atacgtagcc gaactgagag 300

gttgatcggc cacattggga ctgagacacg gcccaaactc tacgggaggc agcagtaggg 360

aatcttccac aatggacgca agtctgatgg agcaacgccg cgtgagtgaa gaaggctttc 420

gggtcgtaaa actctgttgt tggagaagaa tggtcggcag agtaactgtt gtcggcgtga 480

cggtatccaa ccagaaagcc acggctaact acgtgccagc agccgcggta atacgtaggt 540

ggcaagcgtt atccggattt attgggcgta aagcgagcgc aggcggtttt ttaagtctga 600

tgtgaaagcc ctcggcttaa ccgaggaagc gcatcggaaa ctgggaaact tgagtgcaga 660

agaggacagt ggaactccat gtgtagcggt gaaatgcgta gatatatgga agaacaccag 720

tggcgaaggc ggctgtctgg tctgtaactg acgctgaggc tcgaaagcat gggtagcgaa 780

caggattaga taccctggta gtccatgccg taaacgatga atgctaggtg ttggagggtt 840

tccgcccttc agtgccgcag ctaacgcatt aagcattccg cctggggagt acgaccgcaa 900

ggttgaaact caaaggaatt gacgggggcc cgcacaagcg gtggagcatg tggtttaatt 960

cgaagcaacg cgaagaacct taccaggtct tgacatcttt tgatcacctg agagatcagg 1020

tttccccttc gggggcaaaa tgacaggtgg tgcatggttg tcgtcagctc gtgtcgtgag 1080

atgttgggtt aagtcccgca acgagcgcaa cccttatgac tagttgccag cattgagttg 1140

ggcactctag taagactgcc ggtgacaaac cggaggaagg tggggatgac gtcaaatcat 1200

catgcccctt atgacctggg ctacacacgt gctacaatgg atggtacaac gagttgcgag 1260

accgcgaggt caagctaatc tcttaaagcc attctcagtt cggactgtag gctgcaactc 1320

gcctacacga agtcggaatc gctagtaatc gcggatcagc acgccgcggt gaatacgttc 1380

ccgggccttg tacacaccgc ccgtcacacc atgagagttt gtaacacccg aagccggtgg 1440

cgtaaccctt ttagggagcg agccgtctaa ggtgggacaa atgattaggg tgaagtcgta 1500

acaaggtagc cgtaggagaa cc 1522

<210> 12
<211> 1505
<212> DNA
<213> Akkermansia muciniphila ATCC BAA-835T

<400> 12
agagtttgat tctggctcag aacgaacgct ggcggcgtgg ataagacatg caagtcgaac 60

gagagaattg ctagcttgct aataattctc tagtggcgca cgggtgagta acacgtgagt 120

aacctgcccc cgagagcggg atagccctgg gaaactggga ttaataccgc atagtatcga 180

aagattaaag cagcaatgcg cttggggatg ggctcgcggc ctattagtta gttggtgagg 240

taacggctca ccaaggcgat gacgggtagc cggtctgaga ggatgtccgg ccacactgga 300

actgagacac ggtccagaca cctacgggtg gcagcagtcg agaatcattc acaatggggg 360

aaaccctgat ggtgcgacgc cgcgtggggg aatgaaggtc ttcggattgt aaacccctgt 420

catgtgggag caaattaaaa agatagtacc acaagaggaa gagacggcta actctgtgcc 480

```
agcagccgcg gtaatacaga ggtctcaagc gttgttcgga atcactgggc gtaaagcgtg      540
cgtaggctgt ttcgtaagtc gtgtgtgaaa ggcgcgggct caacccgcgg acggcacatg      600
atactgcgag actagagtaa tggagggga accggaattc tcggtgtagc agtgaaatgc      660
gtagatatcg agaggaacac tcgtggcgaa ggcgggttcc tggacattaa ctgacgctga      720
ggcacgaagg ccaggggagc gaaagggatt agatacccct gtagtcctgg cagtaaacgg      780
tgcacgcttg gtgtgcgggg aatcgacccc ctgcgtgccg gagctaacgc gttaagcgtg      840
ccgcctgggg agtacggtcg caagattaaa actcaaagaa attgacgggg acccgcacaa      900
gcggtggagt atgtggctta attcgatgca acgcgaagaa ccttacctgg gcttgacatg      960
taatgaacaa catgtgaaag catgcgactc ttcggaggcg ttacacaggt gctgcatggc     1020
cgtcgtcagc tcgtgtcgtg agatgtttgg ttaagtccag caacgagcgc aacccctgtt     1080
gccagttacc agcacgtgaa ggtggggact ctggcgagac tgcccagatc aactgggagg     1140
aaggtgggga cgacgtcagg tcagtatggc ccttatgccc agggctgcac acgtactaca     1200
atgcccagta cagagggggc cgaagccgcg aggcggagga aatcctaaaa actgggccca     1260
gttcggactg taggctgcaa cccgcctaca cgaagccgga atcgctagta atggcgcatc     1320
agctacggcg ccgtgaatac gttcccgggt cttgtacaca ccgcccgtca catcatggaa     1380
gccggtcgca cccgaagtat ctgaagccaa ccgcaaggag gcagggtcct aaggtgagac     1440
tggtaactgg gatgaagtcg taacaaggta gccgtagggg aacctgcggc tggatcacct     1500
ccttt                                                                 1505
```

## Description

### Technical Field

The present invention relates to an agent for increasing intestinal butyric acid, a food composition for increasing intestinal butyric acid, a proliferation agent for butyric acid-producing bacteria, a food composition for proliferating butyric acid-producing bacteria, a method for increasing intestinal butyric acid, a method for proliferating butyric acid-producing bacteria, and a method for the treatment or prevention of intestinal inflammation, fatty liver, diabetes, colorectal cancer, or obesity and the use of 1-kestose for producing a pharmaceutical preparation for the treatment or prevention of these diseases.

### Background Art

Butyric acid is a short chain fatty acid and is produced by butyric acid-producing bacteria that reside in the intestines in human and animal bodies. Studies in recent years have reported that butyric acid has various physiological effects such as anti-inflammatory effects and colorectal cancer preventing effects and that butyric acid may prevent or ameliorate various diseases.

Based on theses, attempts to use butyric acid have been made for prevention and treatment of diseases and for health promotion. Meanwhile, butyric acid is a substance having a strong bad smell and therefore butyric acid itself is difficult to be added to and used in pharmaceutical preparations and foods. Therefore, attempts to increase the amount of butyric acid in human and animal intestines have been made. For example, Patent Literature 1 discloses a food composition having the effect of increasing the intestinal butyric acid concentration and comprising D-mannitol or D-sorbitol as an active ingredient and Patent Literature 2 discloses an agent for increasing intestinal butyric acid-producing bacteria, comprising Bacillus subtilis cells as an active ingredient.

### Citation List

#### Patent Literature

Patent Literature 1: Japanese Patent Laid-Open No. 2004-49093
Patent Literature 2: Japanese Patent Laid-Open No. 2013-147469

### Summary of the Invention

#### Technical Problem

However, no substance or method capable of effectively increasing intestinal butyric acid has been sufficiently provided and development of such a substance or method has been desired. The present invention was made to solve the problem and an object of the present invention is to provide an agent for increasing intestinal butyric acid, a food composition for increasing intestinal butyric acid, a proliferation agent for butyric acid-producing bacteria, a food composition for proliferating butyric acid-producing bacteria, a method for increasing intestinal butyric acid, and a method for proliferating butyric acid-producing bacteria that are capable of effectively increasing intestinal butyric acid and a method for treatment or prevention of intestinal inflammation, fatty liver, diabetes, colorectal cancer, or obesity using one of these and the use of 1-kestose for producing a pharmaceutical preparation for treatment or prevention of these diseases.

#### Solution to the Problem

The present inventors have found, as a result of diligent studies, that 1-kestose increases the amount and the concentration of intestinal butyric acid and that 1-kestose increases the number of intestinal butyric acid-producing bacteria. Accordingly, based on these findings, the following inventions were completed.

(1) The agent for increasing intestinal butyric acid according to the present invention comprises 1-kestose as an active ingredient.
(2) The agent for increasing intestinal butyric acid according to the present invention is preferably used so that a human or an animal take 0.04 g/kg body weight or more of 1-kestose per day.

(3) The agent for increasing intestinal butyric acid according to the present invention can be used for prevention or treatment of intestinal inflammation, fatty liver, diabetes, colorectal cancer, or obesity.
(4) The food composition for increasing intestinal butyric acid according to the present invention comprises 1-kestose as an active ingredient.
(5) The proliferation agent for butyric acid-producing bacteria according to the present invention comprises 1-kestose as an active ingredient.
(6) The food composition for proliferating butyric acid-producing bacteria according to the present invention comprises 1-kestose as an active ingredient.
(7) The method for increasing intestinal butyric acid according to the present invention comprises the step of increasing the amount or the concentration of butyric acid in intestines in a human or an animal by letting the human or the animal take 1-kestose.
(8) The first aspect of the method for proliferating butyric acid-producing bacteria according to the present invention comprises a step of increasing the number of butyric acid-producing bacteria in intestines in a human or an animal by letting the human or the animal take 1-kestose.
(9) The second aspect of the method for proliferating butyric acid-producing bacteria according to the present invention comprises a step of adding 1-kestose to a medium containing butyric acid-producing bacteria to culture the butyric acid-producing bacteria.
(10) The method for treatment or prevention of intestinal inflammation, fatty liver, diabetes, colorectal cancer, or obesity according to the present invention comprises a step of increasing the amount or the concentration of butyric acid or the number of butyric acid-producing bacteria in intestines in a human or an animal having or being at risk of having intestinal inflammation, fatty liver, diabetes, colorectal cancer, or obesity by letting the human or the animal take 1-kestose.
(11) The use of 1-kestose according to the present invention is use of 1-kestose for producing a pharmaceutical preparation for treatment or prevention of intestinal inflammation, fatty liver, diabetes, colorectal cancer, or obesity.

Advantageous Effects of Invention

1-kestose is an oligosaccharide and a substance that is contained in vegetables such as onion and garlic, and in cereals such as barley and rye and has been present in foods since ancient times. Moreover, no toxicity of 1-kestose has been found in any of mutagenicity, acute toxicity, subchronic toxicity, and chronic toxicity tests. From these, 1-kestose is considered to be very safe (Food processing and ingredients, Vol. 49, No. 12, p. 9, 2014). Moreover, since 1-kestose is highly water-soluble and has a good sweet taste quality similar to sugar, it can be taken easily on a daily basis as it is or as a sweetener or the like and can be easily formulated into various foods, pharmaceutical preparations and the like.

Accordingly, intestinal butyric acid in humans and animals can be easily and effectively increased with little concerns about side effects and safety, according to the present invention. Moreover, according to the present invention, intestinal inflammation, fatty liver, diabetes, colorectal cancer, or obesity can be prevented or treated by increasing intestinal butyric acid in a human or an animal.

Brief Description of the Drawings

Figure 1 is a table illustrating the concentrations and the amounts of short chain fatty acids (acetic acid, isovaleric acid, and valeric acid) in cecal contents in rats (Groups A to E) given 0 to 1.34 g/kg body weight of 1-kestose.
Figure 2 is a set of bar graphs illustrating the concentrations and the amounts of short chain fatty acids (butyric acid, propionic acid, and isobutyric acid) in cecal contents in rats (Groups A to E) given 0 to 1.34 g/kg body weight of 1-kestose.
Figure 3 is a set of bar graphs illustrating the concentrations and the amounts of short chain fatty acids (acetic acid, isovaleric acid, and valeric acid) in cecal contents in rats (Groups A to E) given 0 to 1.34 g/kg body weight of 1-kestose.
Figure 4 (I) is a table illustrating the means, standard deviations, and significant difference of the copy number of 16S rDNA from various microorganisms in cecal contents in rats given no 1-kestose (Group A) and rats given 1-kestose (Group E). Figure 4 (II) is a bar graph illustrating the means and standard deviations of the copy number of the 16S rDNA.

Description of Embodiments

Hereinafter, an agent for increasing intestinal butyric acid, a food composition for increasing intestinal butyric acid, a proliferation agent for butyric acid-producing bacteria, a food composition for proliferating butyric acid-producing bacteria, a method for increasing intestinal butyric acid, a method for proliferating butyric acid-producing bacteria, and a method for treatment or prevention of intestinal inflammation, fatty liver, diabetes, colorectal cancer, or obesity and the use of 1-kestose for producing a pharmaceutical preparation for treatment or prevention of these diseases according to the present invention will be described in detail.

"Butyric acid" is a linear carboxylic acid that is also referred to as butanoic acid or n-butyric acid, and has a molecular formula of $C_4H_8O_2$ and a rational formula of $CH_3(CH_2)_2COOH$. Butyric acid is known to be produced by butyric acid-producing bacteria habitually residing in the intestines in humans and animals.

In the present invention, "butyric acid-producing bacteria" are microorganisms that produce butyric acid. Specific examples of such microorganisms include bacteria included in Clostridium cluster-XIVa such as Eubacterium Hallii, Butyrivibrio crossotus, Coprococcus eutactus, Coprococcus catus, Clostridium symbiosum, and Roseburia cecicola; bacteria included in Clostridium cluster-XVI such as Clostridium innocuum, Eubacterium tortuosum, and Eubacterium cylindroides; bacteria included in Clostridium cluster-I such as Clostridium butyricum, Eubacterium moniliforme, and Clostridium acetobutylicum; bacteria included in Clostridium cluster-IV such as Clostridium sporosphaeroides and Eubacterium sp. A2-207; and bacteria included in Clostridium cluster-XV such as Eubacterium barkeri and Eubacterium limosum.

"1-kestose" is an oligosaccharide that is a trisaccharide composed of 1 molecule of glucose and 2 molecules of fructose. 1-kestose can be obtained by an enzymatic reaction of sucrose as a substrate with an enzyme such as one disclosed in Japanese Patent Laid Open No. 58-201980. Specifically, β-fructofuranosidase is added to a sucrose solution and the enzymatic reaction is performed by leaving the mixture at 37°C to 50°C for around 20 hours to obtain a reaction solution containing 1-kestose. This reaction solution containing 1-kestose is purified by separating 1-kestose and other sugars (glucose, fructose, sucrose, oligosaccharides composed of 4 or more sugars) using the chromatographic separation disclosed in Japanese Patent Laid Open No. 2000-232878 to obtain a high purity 1-kestose solution. 1-kestose can be obtained as crystals by subsequently concentrating this high purity 1-kestose solution and then crystallizing 1-kestose by a crystallization process such as one disclosed in Japanese Patent Publication No. 6-70075.

Moreover, 1-kestose is contained in commercially available fructo-oligosaccharides and therefore these may be used as they are or after separating and purifying 1-kestose from the fructo-oligosaccharides by the aforementioned method. Accordingly, 1-kestose-containing compositions such as oligosaccharides containing 1-kestose may be used as 1-kestose of the present invention. When a 1-kestose-containing composition is used, the purity of the 1-kestose is preferably 80% by mass or more, more preferably 85% by mass or more, and further preferably 90% by mass or more. The "purity" of 1-kestose in the present invention is percent by mass of 1-kestose when taking the total amount of the sugars as 100%.

1-kestose can be used by letting a human or an animal take the 1-kestose. An example of the intake (dose) of 1-kestose is 0.04 g/kg body weight or more per day. Such an intake may be taken once a day or divided into multiple doses. As illustrated in Example 1 described below, such an intake can markedly increase the concentration and the amount of intestinal butyric acid.

1-kestose functions to increase the amount and the concentration of butyric acid in the intestines in humans and animals. Moreover, 1-kestose functions to increase the number of butyric acid-producing bacteria in the intestines in humans and animals. Accordingly, the method for letting a human or animal take 1-kestose by may be any method, as long as it is a method that allows 1-kestose to reach the intestine thereof. Specific examples of the method include letting a human or an animal take 1-kestose as it is or in the form of beverage or food or a pharmaceutical preparation. Other examples include administering 1-kestose from anus directly or through an inserted tube, adding 1-kestose to an enteral nutrition agent and administering it by enteral nutrition via a tube inserted into a gastrointestinal tract such as the stomach or the small intestine, and the like.

In addition, 1-kestose can be used in vitro to proliferate butyric acid-producing bacteria. In this case, for example, a method adding 1-kestose to a medium containing butyric acid-producing bacteria to culture the butyric acid-producing bacteria in predetermined culture conditions suitable for the butyric acid-producing bacterium is available. Specific examples include a method inoculating Clostridium butyricum, when Clostridium butyricum is used as butyric acid-producing bacterium, into a medium containing 2% by weight of 1-kestose as carbon source, 2% by weight of an amino acid solution as nitrogen source, 0.75% by weight of calcium carbonate and anaerobically culturing the butyric acid-producing bacterium at 35 to 37°C for 16 to 24 hours.

Butyric acid has been confirmed to exhibit, in model rats with ulcerative colitis induced by dextran sulfate sodium (DSS), a significant inflammatory repair effect by injection into the intestine. Moreover, it has been confirmed that the oral administration of a spore formulation of Clostridium butyricum M588, a butyric acid-producing bacterium, has a prominent effect of reducing inflammation in the same model rat. Furthermore, butyric acid injection therapy is provided for human ulcerative colitis (Journal of intestinal microbiology, vol. 19(1), p. 1-8, 2005; right column on page 2, Figure 4-1 and 4-2, etc.).

Therefore, intestinal inflammation can be prevented or treated by increasing the concentration or the amount of butyric acid or the number of butyric acid-producing bacteria in the intestines by letting a human or an animal take 1-kestose. Moreover, 1-kestose can be used to produce a pharmaceutical preparation for treatment or prevention of intestinal inflammation.

Here, in the present invention, "intestinal inflammation" means conditions with inflammation in the intestinal tract and includes disease conditions. Examples of diseases developed from intestinal inflammation include inflammatory bowel disease. The term inflammatory bowel disease may narrowly refer ulcerative colitis and Crohn disease or generally refer any enteric inflammatory disease (Integrated handbook of internal medicine (Progress 8), Gastrointestinal Diseases, pp. 320, 1997, Nakayama Shoten Co., Ltd.). The inflammatory bowel disease in the present invention means the latter, namely any inflammatory disease in an intestinal tract. The present invention may preferably be used for the prevention or treatment of intestinal inflammation excluding allergy.

Moreover, oral administration of butyric acid to rats at 0.02 g/kg body weight per day has been shown to suppress fat degeneration and inflammation of the liver due to the intake of high-fat meals, and it normalizes the values of AST (aspartate aminotransferase), ALT (alanine aminotransferase), cholesterol, LDL (low-density lipoprotein) in blood, neutral fat and fasting blood sugar levels, and the insulin resistance index (HOMA-IR) (G. Mattace Raso et al., PLoS ONE, vol. 8, no. 7, e68626, 2013, Abstract, Result, etc.). Therefore, fatty liver can be prevented or treated by increasing the concentration or the amount of butyric acid or the number of butyric acid-producing bacteria in the intestines by letting a human or an animal take 1-kestose. Moreover, 1-kestose can be used to produce a pharmaceutical preparation for treatment or prevention of fatty liver.

Moreover, intraperitoneal administration of butyric acid to a childhood diabetes model rat at 500 mg/kg of body weight per day (in terms of the dose of sodium butyrate) has been shown to decrease β cell death, improves the function and proliferation of β cells, and improves glucose homeostasis (S. Khan et al., Chem. Biol. Interact., vol. 213, p. 1-12, 2014, Abstract, etc.). Therefore, diabetes can be prevented or treated by increasing the concentration or the amount of butyric acid or the number of butyric acid-producing bacteria in the intestines by letting a human or an animal take 1-kestose. Moreover, 1-kestose can be used to produce a pharmaceutical preparation for treatment or prevention of diabetes.

Moreover, by addition of butyric acid to the culture of LIM1215 at final concentration of 1 mmol/L, human colorectal cancer cells have been shown to extend the proliferation time and decrease the proliferation rate (Whitehead RH et al., Gut 27, p. 1457-1463, 1986, SUMMARY, etc.). Furthermore, it has been reported that butyric acid suppresses the development of colorectal cancer by suppressing abnormal proliferation of mucosa epithelial cells in the large intestine, promoting the cell death and differentiation of mutated cells, suppressing the transcription of abnormal proteins, or suppressing the development of aberrant crypt foci (ACF) which is a lesion in the early period of colorectal cancer (Journal of intestinal microbiology, vol. 16(1), p. 35-42, 2002, left column on p.40 and Table 5, etc.). Therefore, colorectal cancer can be prevented or treated by increasing the concentration or the amount of butyric acid or the number of butyric acid-producing bacteria in the intestines by letting a human or an animal take 1-kestose. Moreover, 1-kestose can be used to produce a pharmaceutical preparation for treatment or prevention of colorectal cancer.

Moreover, oral administration of butyric acid at 5 g/kg body weight per day (in terms of the dose of sodium butyrate) has been shown to reduce body weight and the body fat percentage in mice in which obesity has been induced by high-fat meals and has been shown to suppress weight gain and increase of the body fat percentage by eating of high-fat meals in mice that are not obese (Zhanguo Gao et. al., DIABETES, VOL. 58, July 2009, p. 1509-1517; FIG. 1 E and F and FIG. 7 A and B, etc.). Similarly, it has been also shown that increase of body weight and increase of the body fat percentage are suppressed in mice fed on high-fat meals into which 5% by mass of sodium butyrate is mixed (Hua V. Lin et. al., PLoS ONE, Vol. 7, Issue 4, e35240, April 2012, Fig. 1 A, etc.). Therefore, obesity can be prevented or treated by increasing the concentration or the amount of butyric acid or the number of butyric acid-producing bacteria in the intestines by letting take a human or an animal 1-kestose. Moreover, 1-kestose can be used to produce a pharmaceutical preparation for treatment or prevention of obesity.

Examples of specific aspects of the agent for increasing intestinal butyric acid and the proliferation agent for butyric acid-producing bacteria according to the present invention include pharmaceutical preparations and quasi drugs, food additives, and health foods such as supplements.

The dosage form of the pharmaceutical preparations, quasi drugs, and supplements containing 1-kestose are not particularly limited, and a dosage form suitable for the mode of administration can be selected as appropriate. For example, when orally administered, the dosage form may be a solid or liquid dosage form such as a powder, a tablet, a dragee, a capsule, a granule, a dry syrup, a solution, a syrup, a drop, and a health drink.

The aforementioned dosage forms of pharmaceutical preparations, quasi drugs, and supplements can be prepared by methods known to those skilled in the art. For example, for a powder, 800 g of 1-kestose and 200 g of lactose are mixed well and then the mixture is wetted by adding 300 mL of 90% ethanol. Subsequently, the wet powder is granulated, dried with ventilation at 60°C for 16 hours, and then sized to obtain 1000 g of a suitable size of powder (1-kestose content 800 mg/1 g). For tablets, 300 g of 1-kestose, 380 g of powdery reduced starch syrup, 180 g of rice starch, and 100 g of dextrin are mixed well and then the mixture is wetted by adding 300 mL of 90% ethanol. Subsequently, the wet powder is extruded and granulated and then dried with ventilation at 60°C for 16 hours to obtain granules. Then, these granules were sized using a 850 μm sieve, followed by adding 50 g of a sucrose fatty acid ester and mixing with 470 g of the sized granules, and then the mixture was pressed into tablets with a rotary tableting machine (6B-2, manufactured by Kikusui Seisakusho Ltd.) to obtain 5000 tablets having a diameter of 8 mm and a weight of 200 mg (1-kestose content 60 mg/tablet).

Specific embodiments of the food composition for increasing intestinal butyric acid and the food composition for proliferating butyric acid-producing bacteria according to the present invention include, for example, beverages, dairy products, granules for eating, pastes, flavoring agents, retort pouches, baby foods, fermented foods, preserved foods, processed foods such as processed marine products, processed meat products, and processed grain products, food additives, health foods, and animal feed.

1-kestose can be used by being added to various foods and drinks, food additives, and animal feed in their normal production process. Since 1-kestose has a degree of sweetness of 30 and its quality of taste, physical properties, and workability are close to those of sucrose, it can be used like sugar in the production process of various foods and drinks, for example, by replacing a part or all sugar with 1-kestose, and various foods and drinks, food additives, and animal feed can be produced.

The present invention will be described with reference to the Examples below. The technical scope of the present invention is not limited by the features illustrated by these Examples. In the following Examples, compositions containing a predetermined purity of 1-kestose are referred to as "1-kestose".

## EXAMPLES

### <Example 1> Study on amount of intestinal short chain fatty acid upon intake of 1-kestose

(1) Breeding of rats with giving oral intake of 1-kestose

The feeds containing 0, 0.5, 1.0, 2.5, and 5% by mass of 1-kestose were prepared by CLEA Japan, Inc. on commission. The composition of the feeds is described below. 40 SD rats (Japan SLC, Inc.) were divided into 5 groups of 8 animals and designated as Groups A to E. The rats were maintained for 30 days with ad libitum feeding of the feed without 1-kestose to Group A and of the ones with 1-kestose to Groups B to E, respectively. The rats were maintained under conditions at 23 ± 1°C in temperature with 12 hours of light periods (from 8:00 to 20:00) and 12 hours of dark periods (from 20:00 to 8:00).

[Composition (in % by mass) of feeds] Cornstarch 39.7486, milk casein 20, pregelatinized cornstarch 13.2, granulated sugar and/or 1-kestose (99% by mass in purity, B Food Science Co., Ltd.) 10, purified soybean oil 7, cellulose powder 5, mineral mixture 3.5, vitamin mixture 1, L-cystine 0.3, choline bitartrate 0.25, tert-butylhydroquinone 0.0014.

(2) Study on amount of intestinal short chain fatty acid

The rats in each group described in Example 1 (1) were dissected and their ceca were extracted. The contents of the ceca were collected and weighed. The concentrations of short chain fatty acids (butyric acid, propionic acid, isobutyric acid, acetic acid, isovaleric acid, and valeric acid) in the cecal contents were measured with a gas chromatography-mass spectrometer (GC/MS). The amounts of the short chain fatty acids in the cecal contents were calculated from the results of the measurement of the short chain fatty acid concentrations. Subsequently, the means of concentrations and amounts of short chain fatty acids for each group were calculated and shown in bar graphs. The results are illustrated in Figures 1 to 3.

The intake of a drug in rat has been reported to be converted into a corresponding human adult intake by the formula 1 below (Paragraph [0065] in Japanese Patent Laid Open No. 2014-526521, Shannon Reagan-Shaw et. al., The FASEB Journal, Vol. 22, March 2007, p. 659-661). Therefore, the daily intake of 1-kestose in rat was converted into corresponding human adult intake by the formula 1. The results were also set forth in Figure 1.

[Formula 1] daily intake of 1-kestose in human adult (g/kg body weight) = daily intake of 1-kestose in rat (g/kg body weight) × 6/37

As illustrated in Figures 1 to 3, the concentrations of butyric acid were 205.86 μg/g in Group A, 288.09 μg/g in Group B (increase to approximately 1.4 times of that in Group A), 314.59 μg/g in Group C (increase to approximately 1.5 times of that in Group A), 754.17 μg/g in Group D (increase to approximately 3.7 times of that in Group A), and 2005.26 μg/g in Group E (increase to approximately 10 times of that in Group A). Moreover, the amounts of butyric acid

was 781.14 μg in Group A, 1069.37 μg in Group B (increase to approximately 1.4 times of that in Group A), 1107.92 μg in Group C (increase to approximately 1.4 times of that in Group A), 2891.65 μg in Group D (increase to approximately 3.6 times of that in Group A), and 11943.88 μg in E group (increase to approximately 14.5 times of that in Group A). Accordingly, the concentrations and the amounts of butyric acid were markedly increased in any of Groups B to E in comparison with those in Group A.

The concentrations and the amounts of acetic acid were also significantly increased in Groups B, C, D, and E in comparison with those in Group A. On the other hand, the concentrations and the amounts of propionic acid and isovaleric acid were not significantly different in Groups B, C, D, and E in comparison with those in Group A. The concentrations and the amounts of isobutyric acid and valeric acid were significantly decreased in Groups B, C, D, and E in comparison with those in Group A.

From this result, it was revealed that the concentration and the amount of cecal butyric acid were increased in the rats fed with the feed supplemented with 1-kestose. Accordingly, it was revealed that intestinal butyric acid increases upon the intake of 1-kestose by humans and animals. Moreover, it was revealed that preferable intake of 1-kestose in human adult is equal to or more than 0.04 g/kg body weight per day to increase intestinal butyric acid since prominent increase in concentration and amount of butyric acid was found in rats with daily intake of 0.27 g/kg body weight of kestose (Group B).

<Example 2> Study on amount of intestinal microorganism upon intake of 1-kestose

The amounts of the following 4 microorganisms in the cecal contents of rats in Groups A and E described in Example 1(1) were measured by real-time PCR.

1. The genus Bifidobacterium,
2. Clostridium cluster-XIVa and Clostridium cluster-XIVb (a group of butyric acid-producing bacteria),
3. The genus Lactobacillus
4. Akkermansia muciniphila

Specifically, first, the cecal contents of Groups A and E were subjected to crushing treatment by using a bead type crusher "FastPrep FP 100 A" (MP Biomedical). Subsequently, genomic DNA was extracted with the nucleic acid extraction instrument "Magtration (R) System 12GC" and its exclusive reagent "MagDEA (R) DNA 200" (Precision System Science Co., Ltd.) according to the instructions. Using this genomic DNA as a template, , the real-time PCR reagent "SYBR (R) Premix Ex Taq II (Tli RNaseH Plus)" (Takara), and the real-time PCR device "Rotor-Gene Q" (QIAGEN N.V.), real-time PCR was performed to find the 16S rDNA copy number of each microorganism per 1 g of cecal contents. The means and standard deviation of the copy numbers were calculated for each group and illustrated in bar graphs. The results are shown in Figure 4.

The sequences of the primers specific for 16S rDNA of each microorganism are listed below.

1. Bifidobacterium

    Forward primer, GATTCTGGCTCAGGATGAACGC (SEQ ID NO: 1)
    Reverse primer, CTGATAGGACGCGACCCCAT (SEQ ID NO: 2)

2. Clostridium cluster XIVa and XIVb (a group of butyric acid-producing bacteria)

    Forward primer, GAWGAAGTATYTCGGTATGT (SEQ ID NO: 3)
    Reverse primer, CTACGCWCCCTTTACAC (SEQ ID NO: 4)

3. Lactobacillus

    Forward primer, CACAATGGACGMAAGTCTGATG (SEQ ID NO: 5)
    Reverse primer, CGCCACTGGTGTTCTTCCAT (SEQ ID NO: 6)

4. Akkermansia muciniphila

    Forward primer, CAGCACGTGAAGGTGGGGAC (SEQ ID NO: 7)
    Reverse primer, CCTTGCGGTTGGCTTCAGAT (SEQ ID NO: 8)

The standard curve was made from the result of real-time PCR for each microorganism under the same conditions using a plasmid DNA in which a part of the following sequence is incorporated as a template.

1. The genus Bifidobacterium; 16S rDNA sequence (SEQ ID NO: 9) of B. longum subsp. longum JCM 1217T
2. Clostridium clusters-XIVa and -XIVb (a group of butyric acid-producing bacteria); 16S rDNA sequence (SEQ ID NO: 10) of Clostridium clostridioforme JCM1291T
3. The genus Lactobacillus; 16S rDNA sequence (SEQ ID NO: 11) of L. casei JCM 1134T
4. Akkermansia muciniphila; 16S rDNA sequence (SEQ ID NO: 12) of A. muciniphila ATCC BAA-835T

As illustrated in Figure 4, the copy number of 16S rDNA from Clostridium cluster XIV (a group of butyric acid-producing bacteria) per 1 g of the cecal contents was 5071250000 (about 0.5E+10) in Group A and 25287500000 (about 2.5E+10) in Group E. Accordingly, the copy number of 16S rDNA from the group of butyric acid-producing bacteria in Group E was approximately 5 times of that in Group A and significantly increased.

The copy number of 16S rDNA from the genus Bifidobacterium conventionally known to be increased in the intestines by intake of kestose (for example, Japanese Patent No. 4669235) was also significantly increased in Group E in comparison with that in Group A. Meanwhile, the copy numbers of 16S rDNA from Lactobacillus and Akkermansia muciniphila did not have significant difference between Group E and Group A.

From this result, it was revealed that the number of cecal butyric acid-producing bacteria was increased in the rats fed with feed with 1-kestose. Accordingly, it was revealed that intestinal butyric acid-producing bacteria proliferate upon intake of 1-kestose by humans and animals.

## Claims

1. An agent for increasing intestinal butyric acid, comprising 1-kestose as an active ingredient.

2. The agent for increasing intestinal butyric acid according to claim 1, wherein the agent is used to let a human or an animal take 0.04 g/kg body weight or more of 1-kestose per day.

3. The agent for increasing intestinal butyric acid according to claim 1 or 2, wherein the agent is used for prevention or treatment of intestinal inflammation, fatty liver, diabetes, colorectal cancer, or obesity.

4. A food composition for increasing intestinal butyric acid, comprising 1-kestose as an active ingredient.

5. A proliferation agent for butyric acid-producing bacteria, comprising 1-kestose as an active ingredient.

6. A food composition for proliferating butyric acid-producing bacteria, comprising 1-kestose as an active ingredient.

7. A method for increasing intestinal butyric acid, comprising the step of increasing the amount or the concentration of butyric acid in intestines in a human or an animal by letting the human or the animal take 1-kestose.

8. A method for proliferating butyric acid-producing bacteria, comprising the step of increasing the number of butyric acid-producing bacteria in intestines in a human or an animal by letting the human or the animal take 1-kestose.

9. A method for proliferating butyric acid-producing bacteria, comprising a step of adding 1-kestose to a medium containing butyric acid-producing bacteria to culture the butyric acid-producing bacteria.

**10.** A method for treatment or prevention of intestinal inflammation, fatty liver, diabetes, colorectal cancer, or obesity, comprising a step of increasing the amount or the concentration of butyric acid or the number of butyric acid-producing bacteria in intestines in a human or an animal having or being at risk of having intestinal inflammation, fatty liver, diabetes, colorectal cancer, or obesity by letting take the human or the animal 1-kestose.

**11.** Use of 1-kestose for producing a pharmaceutical preparation for treatment or prevention of intestinal inflammation, fatty liver, diabetes, colorectal cancer, or obesity.

# Figures

[Figure 1]

| | Daily feed intake (g/g body weight) | Daily 1-kestose intake (g/kg body weight) | Daily 1-kestose intake of human adult (g/kg body weight) | Weight of cecal contents (g) | Cncentration and amount of short chain fatty acids | | | | | | | |
|---|---|---|---|---|---|---|---|---|---|---|---|---|
| | | | | | | | Butyric acid | Propionic acid | Isobutyric acid | Acetic acid | Isovaleric acid | Valeric acid |
| Group A (without 1-kestose) | 0.05 | 0.00 | 0.00 | 3.83 | Concentration (μg/g cecal contents) | Mean | 205.86 | 977.07 | 81.77 | 2130.79 | 55.83 | 93.14 |
| | | | | | | Standard deviation | 46.09 | 184.52 | 14.23 | 283.45 | 23.60 | 24.13 |
| | | | | | Amount (μg) | Mean | 781.14 | 3747.81 | 311.94 | 8043.63 | 249.52 | 365.59 |
| | | | | | | Standard deviation | 378.82 | 1891.32 | 148.94 | 3600.72 | 270.66 | 208.04 |
| Group B (with 0.5% 1-kestose) | 0.05 | 0.27 | 0.04 | 3.71 | Concentration (μg/g cecal contents) | Mean | 288.09 | 988.82 | 64.18 | 2542.87 | 37.05 | 80.14 |
| | | | | | | Standard deviation | 108.74 | 174.39 | 8.64 | 499.18 | 8.68 | 18.89 |
| | | | | | Amount (μg) | Mean | 1069.37 | 3732.56 | 241.66 | 9435.67 | 142.02 | 306.51 |
| | | | | | | Standard deviation | 487.93 | 1286.15 | 78.89 | 2950.95 | 60.08 | 125.22 |
| Group C (with 1% 1-kestose) | 0.05 | 0.54 | 0.09 | 3.49 | Concentration (μg/g cecal contents) | Mean | 314.59 | 938.04 | 43.87 | 2530.36 | 24.54 | 70.19 |
| | | | | | | Standard deviation | 69.75 | 80.63 | 12.38 | 351.37 | 7.80 | 22.48 |
| | | | | | Amount (μg) | Mean | 1107.92 | 3291.69 | 154.62 | 8870.32 | 85.83 | 248.89 |
| | | | | | | Standard deviation | 352.03 | 824.25 | 61.06 | 2370.66 | 33.07 | 111.63 |
| Group D (with 2.5% 1-kestose) | 0.05 | 1.34 | 0.22 | 3.81 | Concentration (μg/g cecal contents) | Mean | 754.17 | 980.09 | 29.35 | 2616.78 | 20.93 | 57.04 |
| | | | | | | Standard deviation | 358.70 | 200.67 | 12.29 | 683.43 | 9.68 | 19.41 |
| | | | | | Amount (μg) | Mean | 2891.65 | 3789.36 | 114.02 | 10163.86 | 81.73 | 218.62 |
| | | | | | | Standard deviation | 1543.92 | 1313.64 | 62.06 | 4075.26 | 48.66 | 99.85 |
| Group E (with 5% 1-kestose) | 0.05 | 2.71 | 0.44 | 5.73 | Concentration (μg/g cecal contents) | Mean | 2005.26 | 778.52 | 36.79 | 3927.02 | 36.18 | 48.72 |
| | | | | | | Standard deviation | 472.42 | 273.22 | 12.38 | 1213.93 | 8.53 | 17.01 |
| | | | | | Amount (μg) | Mean | 11943.88 | 4568.92 | 208.41 | 22739.42 | 207.21 | 268.23 |
| | | | | | | Standard deviation | 5159.76 | 2438.90 | 96.56 | 10336.24 | 81.12 | 111.21 |

[Figure 2]

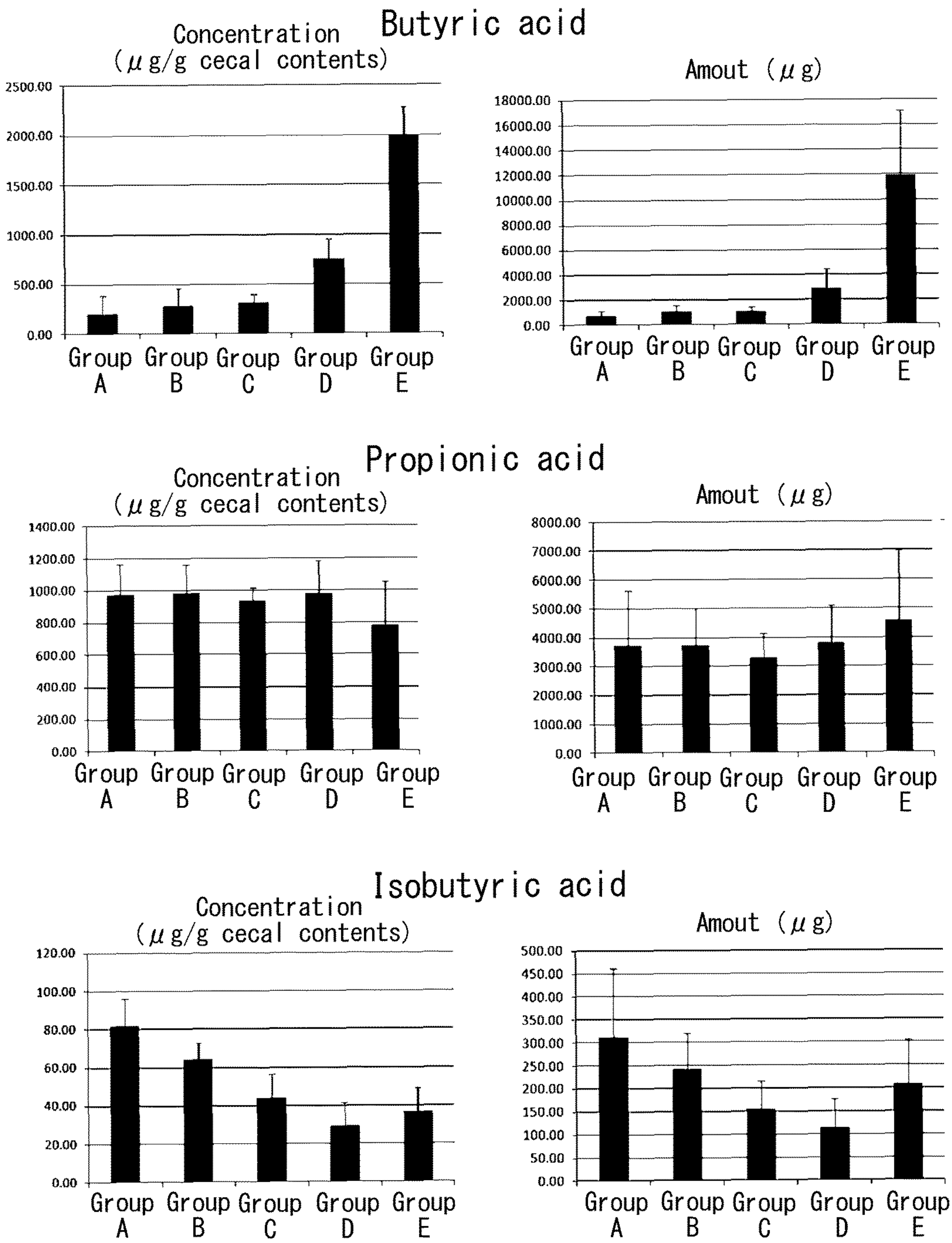
Butyric acid
Concentration (μg/g cecal contents)
Amout (μg)
Group A
Group B
Group C
Group D
Group E
Propionic acid
Concentration (μg/g cecal contents)
Amout (μg)
Isobutyric acid
Concentration (μg/g cecal contents)
Amout (μg)

[Figure 3]

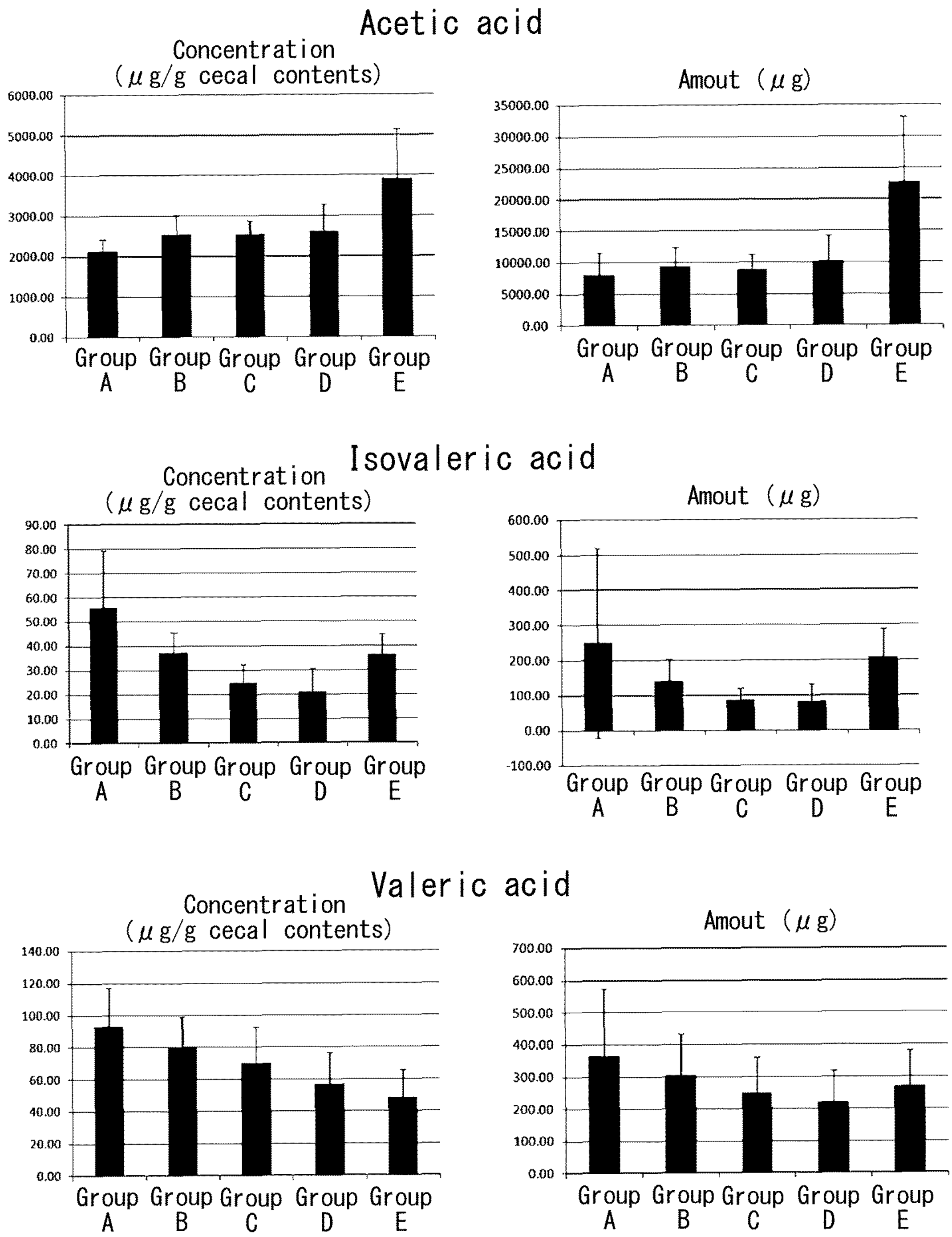
Acetic acid
Concentration (μg/g cecal contents)
6000.00
5000.00
4000.00
3000.00
2000.00
1000.00
0.00
Amout (μg)
35000.00
30000.00
25000.00
20000.00
15000.00
10000.00
5000.00
0.00
Isovaleric acid
Concentration (μg/g cecal contents)
90.00
80.00
70.00
60.00
50.00
40.00
30.00
20.00
10.00
0.00
Amout (μg)
600.00
500.00
400.00
300.00
200.00
100.00
0.00
-100.00
Valeric acid
Concentration (μg/g cecal contents)
140.00
120.00
100.00
80.00
60.00
40.00
20.00
0.00
Amout (μg)
700.00
600.00
500.00
400.00
300.00
200.00
100.00
0.00
Group A
Group B
Group C
Group D
Group E

[Figure 4]

(I)

| | | Bifidobacterium | Clostridium clusters-XIVa, b | Lactobacillus | Akkermansia muciniphila |
|---|---|---|---|---|---|
| Group A | Mean | 11825000 | 5071250000 | 644375000 | 1208875000 |
| | Standard deviation | 0.13 | 0.22 | 0.30 | 0.41 |
| Group E | Mean | 66648750000 | 25287500000 | 1314125000 | 1307571429 |
| | Standard deviation | 0.54 | 0.16 | 0.49 | 0.38 |
| | TTEST | 3.14235E-11 | 3.02464E-06 | 0.451247075 | 0.848766779 |
| | Significant difference | ○ | ○ | × | × |

Unit: Copy number of 16S rDNA/g cecal contents

(II)

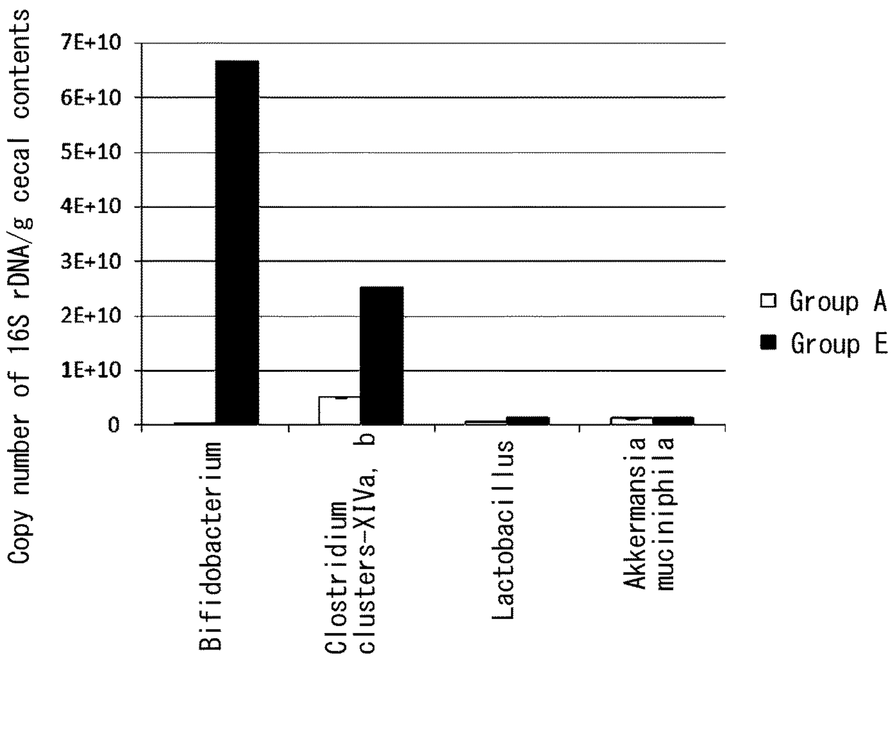

## INTERNATIONAL SEARCH REPORT

International application No. PCT/JP2017/010066

**A. CLASSIFICATION OF SUBJECT MATTER**

*A61K31/702*(2006.01)i, *A23L33/125*(2016.01)i, *A61P1/04*(2006.01)i, *A61P3/04*(2006.01)i, *A61P3/10*(2006.01)i, *A61P35/00*(2006.01)i, *A61P43/00*(2006.01)i, *C12N1/20*(2006.01)i

According to International Patent Classification (IPC) or to both national classification and IPC

**B. FIELDS SEARCHED**

Minimum documentation searched (classification system followed by classification symbols)
A61K31/00-33/44, A23L33/00-33/29, A61P1/00-43/00, C12N1/00-7/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| A | SUZUKI, Nobuyuki et al., Superiority of 1-kestose, the Smallest Fructo-oligosaccharide, to a Synthetic Mixture of Fructo-oligosaccharides in the Selective Stimulating Activity on Bifidobacteria, Bioscience and Microflora, 2006, Vol. 25, No. 3, p. 109-116, ISSN 1349-8355, particularly, p.110, left column, lines 9 - 29, p.111, rigth column, lines 13 - p.112, left column, lines 5, Table 1 | 1-11 |
| A | JP 2007-512840 A (The Iams Co.), 24 May 2007 (24.05.2007), claims; paragraph [0030] & US 2005/0118299 A1 claims; paragraph [0031] & WO 2005/053427 A1 | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 April 2017 (24.04.17) | 16 May 2017 (16.05.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2017/010066

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-049093 A (Meiji Milk Products Co., Ltd.), 19 February 2004 (19.02.2004), paragraph [0001] (Family: none) | 1-11 |
| A | JP 2013-147469 A (Calpis Co., Ltd.), 01 August 2013 (01.08.2013), paragraph [0004] (Family: none) | 1-11 |
| A | JP 2006-321786 A (The Hokuren Federation of Agricultural Cooperations, Japan), 30 November 2006 (30.11.2006), claims & EP 1878738 A1 claims & WO 2006/115136 A1 | 1-11 |
| A | JP 2005-306781 A (The Hokuren Federation of Agricultural Cooperations, Japan), 04 November 2005 (04.11.2005), claims (Family: none) | 1-11 |
| A | KIHARA, Minoru et al., Production of short-chain fatty acids and gas from various oligosaccharides by gut microbes of carp (Cyprinus carpio L.) in micro-scale batch culture, Comparative Biochemistry and Physiology - Part A, 2002, Vol. 132, No. 2, p. 333-340, ISSN 1095-6433, particularly, Abstract | 1-11 |
| A | MURAI, Kenji et al., Effect of Oral Administration to Rats of Various Undigestible Saccharides on Fecal pH, Water Contents and Enzyme Activities, Bifidobacteria and Microflora, 1994, Vol. 13, No. 2, p. 91-98, ISSN 1884-512, particularly, Abstract | 1-11 |
| A | KILIAN, Stephanus et al., The effects of the novel bifidogenic trisaccharide, neokestose, on the human colonic microbiota, World Journal of Microbiology and Biotechnology, 2002, Vol. 18, No. 7, p. 637-644, ISSN 1573-0972, particularly, Summary | 1-11 |
| A | BUYUKTORTOP, Asuman Sahin et al., Determination and Comparison of Interaction Between Fructo-oligosaccharide Components and Lactobacillus acidophilus, SDU Journal of Science (E-Journal), 2012, Vol. 7, No. 1, p. 15-22, ISSN 1306-7575, particularly, Abstract | 1-11 |

Form PCT/ISA/210 (continuation of second sheet) (January 2015)

INTERNATIONAL SEARCH REPORT

International application No. PCT/JP2017/010066

C (Continuation). DOCUMENTS CONSIDERED TO BE RELEVANT

| Category* | Citation of document, with indication, where appropriate, of the relevant passages | Relevant to claim No. |
|---|---|---|
| A | Yasunori FUKUMORI et al., "1-Kestose no Kinosei ni Tsuite", Proceedings of the Research Society of Japan Sugar Refineries' Technologists, 2004, vol.52, pages 13 to 18, ISSN 0370-9841, particularly, Results, Study | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004049093 A
- JP 2013147469 A
- JP 58201980 A
- JP 2000232878 A
- JP 6070075 A
- JP 2014526521 A
- JP 4669235 B


### Non-patent literature cited in the description


- *Food processing and ingredients,* 2014, vol. 49 (12), 9
- *Journal of intestinal microbiology,* 2005, vol. 19 (1), 1-8
- Integrated handbook of internal medicine (Progress 8). Gastrointestinal Diseases. Nakayama Shoten Co., Ltd, 1997, 320
- **G. MATTACE RASO et al.** *PLoS ONE,* 2013, vol. 8 (7), e68626
- **S. KHAN et al.** *Chem. Biol. Interact.,* 2014, vol. 213, 1-12
- **WHITEHEAD RH et al.** *Gut,* 1986, vol. 27, 1457-1463
- *Journal of intestinal microbiology,* 2002, vol. 16 (1), 35-42
- **ZHANGUO GAO.** *DIABETES,* July 2009, vol. 58, 1509-1517
- **HUA V. LIN.** *PLoS ONE,* April 2012, vol. 7 (4), e35240
- **SHANNON REAGAN-SHAW.** *The FASEB Journal,* March 2007, vol. 22, 659-661